(19) Europäisches Patentamt
European Patent Office
Office européen des brevets

(11) Numéro de publication: **0 089 302**
**B1**

# (12) FASCICULE DE BREVET EUROPEEN

(45) Date de publication du fascicule du brevet: **11.09.85**

(51) Int. Cl.[4]: **C 07 C 49/675**, C 07 C 45/45, C 11 B 9/00, A 61 K 7/46

(21) Numéro de dépôt: **83430007.1**

(22) Date de dépôt: **24.02.83**

## (54) Dérivés substitués polyalkylés des indanones-1, procédé de préparation et applications dans des parfums.

(30) Priorité: **11.03.82 FR 8204293**

(43) Date de publication de la demande: **21.09.83 Bulletin 83/38**

(45) Mention de la délivrance du brevet: **11.09.85 Bulletin 85/37**

(84) Etats contractants désignés: **BE CH DE GB IT LI NL**

(56) Documents cités:
**US - A - 2 815 381**
**US - A - 2 815 382**

**HELVETICA CHIMICA ACTA, vol. 48, fasc. 7, no. 159, 1965, pages 1476-1485, Basel CH; F.H. MARQUARDT: "Friedel-Crafts-Reaktionen mit aromatischen Äthern; 1. Mitteilung. Die Herstellung von Alkoxy-indanonen-(1)"**

**Le dossier contient des informations techniques présentées postérieurement au dépôt de la demande et ne figurant pas dans le présent fascicule.**

(73) Titulaire: **P. ROBERTET & Cie Société Anonyme dite, 37, Avenue Sidi-Brahim, F-06333 Grasse (FR)**

(72) Inventeur: **Joulain, Daniel, Cybele residences Chemin de la Cavalerie, F-06130 Grasse (FR)**

(74) Mandataire: **Azais, Henri et al, c/o CABINET BEAU DE LOMENIE 14, rue Raphael, F-13008 Marseille (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

**Description**

La présente invention a pour objet de nouveaux produits qui sont des dérivés substitués polyalkylés des indanones-1, des procédés de préparation de ces produits et des applications de ces produits dans des compositions de parfum.

Les produits à odeur musquée sont très recherchés en parfumerie pour diverses utilisations telles que la préparation d'extraits, d'eaux de toilette, de produits cosmétiques ou d'hygiène corporelle, de produits d'entretien ménager etc. ...

Le musc naturel extrait du chevrotain porte-musc est très onéreux et les chimistes ont cheché à mettre au point des produits synthétiques dégageant une odeur la plus proche possible de celle du musc naturel. Un nombre très important de produits à odeur de musc ont été synthétisés, mais quelques uns seulement sont exploités à l'échelle industrielle.

Parmi ceux-ci, un certain nombre possèdent une fonction cétonique portée par un système polycyclique comportant un noyau aromatique. On connaît, notamment et on utilise pour leur odeur musquée les dérivés ci-après des indanes:

— l'acétyl-7 diméthyl-3,3 tertio butyl-5 indane.
— l'acétyl-6 hexaméthyl-1,1,2,3,3,5 indane.
— l'acétyl-6 isopropyl-1, tétraméthyl-2,3,3,5 indane.

Ces dérivés sont connus respectivement sous les noms de Célestolide, Phantolide et Traséolide. Ce dernier est décrit dans le brevet français 78/05.455 (2 460 913)-NAARDEN.

On connaît également un dérivé de la tétraline qui est:

— l'acétyl-7 hexaméthyl-1,1,3,4,4,6 tétrahydro-1,2,3,4 naphthalène, connu sous le nom de Tonalide.

Tous ces produits connus comportent une fonction cétone faisant partie d'un substituant acétyl et sont des indanes ou une tétraline comportant un noyau hexagonal accolé à un noyau pentagonal ou hexagonal.

On a essayé de synthétiser des hydrocarbures polycycliques dans lesquels la fonction cétone est fixée directement sur un cycle.

La publication J. Agr. Food Chem. 1967, 15, 6, fait mention de deux dérivés substitués des indacènes qui sont la tétraméthyl-5,5,7,7 tétrahydro-3,5,7,7 indacène (2H) one-1 et la tétraméthyl-5,5,8,8-hexahydro-2,3,5,6,7,8 benz(f) indène one-1.

Le brevet US-2 815 381 (The GIVAUDAN Corporation) décrit des dérivés polyalkylés des hydrindacénones-3 asymétriques et des hydrindacénones-1 symétriques parmi lesquels la tétraméthyl-5,5,7,7 hydrindacénone-1 symétrique, tous ces produits ayant une odeur de musc.

Le brevet US-2 815 382 (The GIVAUDAN Corporation) décrit des dérives polyalkylés des tétrahydronaphtindanones-1 et des tétrahydronaphtindanones-3, parmi lesquels la tétraméthyl-5,5,8,8, tétrahydro $\beta$ naphtindanone-1.

Ces derniers produits dégagent une odeur musquée faible.

L'objectif de l'invention est de procurer de nouveaux produits synthétiques dérivés substitués polyalkylés des indanones présentant une odeur musquée forte et très voisine de celle du musc naturel.

La demanderesse a découvert que ce résultat est atteint au moyen de nouveaux dérivés polyalkylés des indanones-1 qui comportent un système polycyclique composé d'un premier noyau benzénique auquel est accolé un deuxième moyau pentagonal portant une fonction cétone en position 1, aucun substituant en position 2 et un seul radical méthyl en position 3 et comportant un troisième noyau accolé au noyau benzénique qui est soit un noyau pentagonal symétrique du deuxième noyau (dans ce cas les produits sont des dérivés des hydro(s)indacène (2H)one-1), soit un noyau hexagonal (dans ce cas les produits sont des dérivés des hydro benz (f) indènes (1H)one-1).

Dans les deux cas, le troisième noyau comporte au moins trois substituants alkylés pris parmi le méthyle, l'éthyle ou le propyle, normal ou iso.

Au cours des nombreux essais qui ont été réalisé, la demanderesse a déterminé que le groupement constitué par une fonction cétone en position 1, aucun substituant en position 2 et un seul radical méthyle en position 3 était déterminant pour obtenir des produits qui dégagent une forte odeur musquée très voisine de l'odeur typique du musc naturel.

La demanderesse a déterminé que la présence d'un substituant méthyle en position 2 avec ou sans un substituant méthyle en position 3, aboutissait à un produit de synthèse sans odeur utilisable. De ême, elle a déterminé que la présence de deux substituants méthyle en position 3 conduisait à un produit de synthèse sans odeur utilisable.

Enfin la demanderesse a établi que la présence de substituants alkyles sur le troisième noyau était importante pour la qualité odoriférante du produit et que seuls les dérivés comportant au moins trois substituants méthyle, éthyle ou propyle fixés au troisième noyau présentant un intérêt dans l'industrie des parfums.

Compte tenu des résultats qui précèdent l'invention a pour objet, à titre de produits nouveaux, les dérivés substitués polyalkylés des indanones-1 appartenant à la famille de substances synthétiques définies par la formule générale:

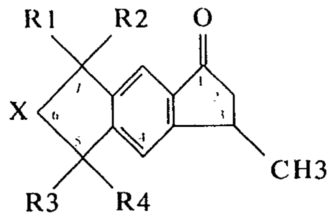

dans laquelle R1, R2, R3, R4 désignent séparément un hydrogène ou un radical alkyle ayant un à trois atomes de carbone, dans laquelle X représente un groupe $-CHR_5-$ ou un groupe $-CHR_5-CHR_6-$, dans lesquels R5 et R6 représentant séparément un hydrogène ou un radical méthyle et dans laquelle au moins trois des radicaux R1, R2, R3, R4, R5 et R6 sont des radicaux alkyles, lesquels dérivés comportant tous une fonction cétone en position 1, aucun substituant en position 2, un seul groupe méthyl en position 3 et un noyau pentagonal ou hexagonal, symétrique du noyau portant la fonction cétone, comportant au moins trois substituants alkyle.

Plus particulièrement, l'invention a pour objet une première famille de nouveaux produits chimiques qui sont des dérivés polyalkylés de l'hydro(s) indacène (2H) one-1 définis par la formule générale:

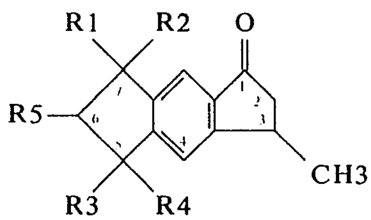

dans laquelle R1, R2, R3, R4 représentent un hydrogène ou un radical alkyle ayant un à trois atomes de carbone, R5 représente un hydrogène ou un radical méthyle et trois au moins des radicaux R1, R2, R3, R4, R5 sont des radicaux alkyle.

L'invention a également pour objet une deuxième famille de nouveaux produits chimiques qui sont des dérivés polyalkylés de l'hydro benz(f) indène (1H)-one-1, définis par la formule générale:

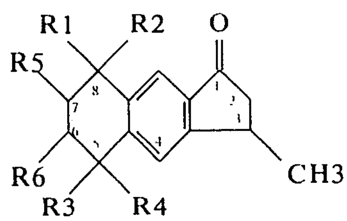

dans laquelle R1, R2, R3, R4 représentent un hydrogène ou un radical alkyle ayant un à trois atomes de carbone, R5, R6, représentent un hydrogène ou un radical méthyle et trois au moins des radicaux R1, R2, R3, R4, R5, R6 sont des radicaux alkyle.

L'invention a également pour objet de nouveaux procédés pour la préparation des dérivés substitués selon l'invention.

On connaît divers procédés de synthèse de dérivés substitués des indanones-1 qui pourraient être utilisés pour préparer les dérivés selon l'invention. Ces procédés connus nécessitent plusieurs étapes et sont peu économiques.

On connaît un procédé permettant de préparer diverses indanones-1 en faisant réagir directement un dérivé aromatique avec un acide éthylénique -$\alpha,\beta$ en présence d'acide polyphosphorique (Cf. article F. H. Marquart dans Helv. Chem. Acta 1965, 48, 1476).

Ce procédé d'annélation connu a déjà été utilisé dans le cas de dérivés comportant un cycle aromatique suffisamment réactif vis à vis de réactions de substitution électrophile aromatique. Dans toutes les applications connues, les substances utilisées comportent toujours un cycle aromatique activé par la présence de substituants alcoxyles, ces derniers agissant par effet électrodonneur mésomère.

Or la préparation de dérivés selon l'invention nécessite que l'on parte d'un dérivé aromatique comportant un cycle aromatique substitué par des radicaux alkyle et, dans ce cas, l'effet activant électrodonneur des substituants sur le cycle aromatique est du type inductif, dont la force est très atténuée par rapport à celle de l'effet mésomère des substituants alcoxyles.

La demanderesse a découvert qu'en se plaçant dans des conditions opératoires appropriées, on

pouvait compenser de façon déterminante, la faible réactivité des composés aromatiques comportant des substituants alkyle et étendre à ceux-ci le domaine d'application du procédé d'annélation connu consistant à faire réagir sur ceux-ci un acide éthylénique $-\alpha,\beta$ en présence d'acide polyphosphorique.

De plus la demanderesse a apporté une amélioration au procédé connu. Elle a en effet découvert que, pour réaliser la condensation, il était avantageux d'utiliser non pas des acides éthyléniques $-\alpha,\beta$ mais des esters méthyliques ou éthyliques de ces acides afin de faciliter la purification du produit résultant de la réaction.

Les procédés selon l'invention consistent à soumettre un dérivé aromatique connu pris dans le groupe des hydrocarbures polyalkylés indanique ou tétraliniques à une réaction de cycli-alkylacylation par un acide éthylénique $-\alpha,\beta$ ou, de préference, oar un ester méthylique ou éthylique d'un acide éthylénique $-\alpha,\beta$ en présence d'acidepolyphosphorique. La réaction a lieu dans un réacteur à une température comprise entre 90°C et 150°C et, de préference, entre 120°C et 125°C si l'on utilise un ester d'acide éthylénique et entre 130°C et 135°C si l'on utilise un acide éthylénique.

On rappelle que les hydrocarbures polyalkylés indaniques ou polyalkyl-indanes connus sont définis par la formule générale:

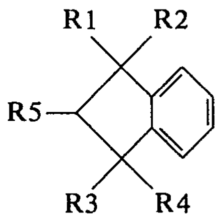

dans laquelle R1, R2, R3, R4, R5 sont des radicaux H ou alkyle.

Pour la préparation des dérivés substitués selon l'invention, ou utilise des polyalkyl-indanes dans lesquels R5 = H ou CH3 et R1, R2, R3, R4 représentent séparément H ou un radical alkyle ayant un à trois atomes de carbone.

La plupart des dérivés polyalkylés indaniques sont connus et on sait les préparer industriellement.

Par exemple le pentaméthyl-1,1,2,3,3 indane et le tétraméthyl-1,1,3,3, indane que lon a utilisé au cours des exemples décrits ci-après, peuvent être préparés selon un procédé décrit par D. B. Spoelstra et Col, dans la publication Rec. Trav. Chim. Pays-Bas, 1963, 82, 1100.

Les hydrocarbures polyalkylés tétraliniques ou polyalkyl tétrahydronaphtalènes sont définis par la formule:

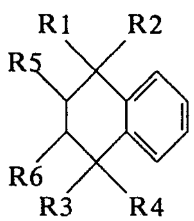

dans laquelle R1, R2, R3, R4, R5, R6 sont séparément H ou un radical alkyle.

Pour la mise en oeuvre du procédé selon l'invention, on utilise les dérivés dans lesquels R5 et R6 représentent séparément H ou CH3 et R1, R2, R3, R4 représentent H ou un radical méthyle, éthyle, propyle ou isopropyle.

On sait préparer ces dérivés aromatiques. Par exemple le tétraméthyl-1,1,4,4, tétrahydro-1,2,3,4 naphtalène que l'on utilise dans les exemples ci-après, peut être préparé par la méthode décrite par G. BADDELEY et M. GORDON dans J. Chem. Soc. 1958, 4379.

Exemple No. 1

Préparation de l'hexaméthyl-3,5,5,6,7,7 tétrahydro-3,5,6,7(s) indacène (2H)one-1

Dans un réacteur de six litres on verse 4500 g d'acide polyphosphorique ($d^{25} = 2,0$ minimum). On chauffe sous agitation mécanique jusqu'à la température de 132° ±3°C. On verse progressivement en 20 à 30 minutes une solution préchauffée à 50°C de 240 g d'acide crotonique et de 470 g de pentaméthyl-1,1,2,3,3 indane. Durant toute la durée d'addition et pendant les 45 minutes suivantes, on maintient les mêmes conditions de température et d'agitation. A l'issue de cette période, on refroidit rapidement la masse réactionnelle à 70°C environ et on la verse sur de la glace pilée en appliquant une agitation énergique. Après décantation de la phase organique et extraction de la phase aqueuse au toluène (2 x 1000 ml), les phases organiques sont réunies et lavées à neutralité au moyen d'une solution aqueuse de carbonate de sodium à 10% puis à l'eau.

Après élimination du toluène sous pression réduite, on obtient 572 g de produit brut que l'on soumet à une première purification dans un distillateur de type »boule à boule«. On recueille 454 g de distillat ayant un point d'ébullition inférieur à 200° C sous une pression de 13,33 Pa. Une seconde purification de ce distillat est effectuée dans un appareil de distillation équipé d'une colonne de Vigreux de 50 cm de longueur. On obtient ainsi, d'une part, 240 g de pentaméthyl-1,1,2,3,3, indane qui a un point d'ébullition de 80—85° C sous 266,66 Pa. Celui-ci constitue le résidu de celui que l'on a introduit dans le réacteur et qui n'a pas réagi. Il est réutilisable dans une nouvelle réaction.

On obtient d'autre part 180 g d'un produit ayant un point d'ébullition de 140—142° C sous 13,33 Pa et un indice de réfraction $n_{20}^{D}$ = 1,551 et qui a été identifié comme étant: l'hexaméthyl-3,5,5,6,7,7 tétrahydro-3,5,6,7(s) indacène (2H)one-1.

Le poids de pentaméthyl-1,1,2,3,3 indane transformé est donc de 230 g et le rendement moléculaire par rapport à la quantité de pentaméthyl indane transformé est de 57%.

La figure 1 représente le spectre d'absorption infrarouge du produit obtenu qui a été indentifié comme étant un mélange de deux isomères qui diffèrent l'un de l'autre par la position relative dans l'espace de deux radicaux méthyle en position 3 et 6. Le mélange des deux isomères peut être utilisé tel quel dans les parfums et compositions de parfums. Par recristallisation dans l'alcol éthylique, on a isolé un isomère pur ayant un point de fusion de 110° C. Les figures 2 et 3 représentent respectivement le spectre d'absorption infrarouge et le spectre de résonance magnétique nucléaire (RMN) à 90 MHz de cet isomère pur.

Le schéma de la réaction obtenue dans cet exemple est le suivant:

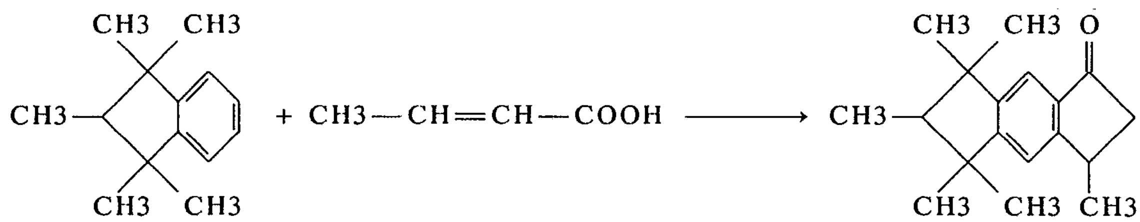

pentaméthyl-1,1,2,3,3, indane + acide crotonique

⟶ hexaméthyl-3,5,5,6,7,7, tétrahydro-3,5,6,7(s)indacène(2H)one-1.

## Exemple No. 2

Dans cet exemple, le dérivé aromatique de départ et le produit final obtenu sout les mêmes que ceux de l'exemple No. 1. L'acide crotonique est remplacé par un ester qui est le crotonate de méthyl ou d'éthyle. A un mélange de 1140 g d'acide polyphosphorique et de 113 g de pentaméthyl-1,1,2,3,3 indane, agité et maintenu constamment à 125° ± 3° C, on ajoute progressivement en deux heures 120 g de crotonate de méthyle. On agite à cette température pendant trois heures, puis on refroidit à 70° C et on procède aux mêmes traitements que dans l'exemple No. 1.

Finalement, on sépare 14 g de pentaméthyl-1,1,2,3,3 indane qui n'a pas réagi et qui est recyclable et 97 g d'hexaméthyl-3,5,5,6,7,7 tétrahydro-3,5,6,7 (s) indacène (2H) one-1.

Le rendement moléculaire est de 71,9% par rapport au pentaméthyl indane transformé.

En remplaçant le crotonate de méthyle par une quantité équivalente de crotonate d'éthyle, on obtient un résultat similaire.

Le produit obtenu dans ces deux exemples dégage une odeur de musc très puissante, se rapprochant beaucoup de l'odeur du musc naturel.

## Exemple No. 3

### Préparation de pentaméthyl-3,5,5,7,7, tétrahydro-3,5,6,7(s) indacène (2H) one-1

On se place dans les mêmes conditions opératoires que dans l'exemple 1. On utilise 4500 g d'acide polyphosphorique, 290 g d'acide crotonique et 450 g de tétraméthyl-1,1,3,3 indane. On récupère 98 g de tétraméthyl-1,1,3,3 indane inchangé, que l'on peut recycler et 300 g d'un produit ayant un point d'ébullition de 136° —138° C sous 13,33 Pa et un point de fusion de 83—84° C. Le spectre d'absorption infrarouge présente une bande principale caractéristique et très forte à 1710 ondes $cm^{-1}$. Le rendement moléculaire est de 61,3% par rapport au tétraméthyl-1,1,3,3 indane transformé.

Le spectre RMN (200 MHz, CDCl3) présente quatre singluets à 1,32, 1,33, 1,34 et 1,35 ppm (quatre groupes méthyl geminés); un doublet à 1,42 ppm (J = 7,4 Hz; méthyle en position 3); un singulet à 1,98 ppm (groupe CH2 en 6); deux doublets de doublets centrés à 2,29 ppm (J = 3,6 et 19 Hz) et à 2,96 ppm (J = 7,6 et 19 Hz) attribués au groupe CH2 en 2; un multiplet complexe centré à 3,41 ppm (J = 3,6; 7,6 et 7,4 Hz, proton tertiaire en 3) et deux singulets à 7,25 et 7,54 ppm (deux protons aromatiques isolès). On a identifié le produit obtenu comme étant du pentaméthyl-3,5,5,7,7 tétrahydro-3,5,6,7(s)indacène(2H)one-1. Ce produit dégage une odeur de musc puissante, légèrement plus atténuée et plus douce que celle du produit obtenu dans les exemples 1 et 2 et rappelant beaucoup celle des oxa-muscs macrocycliques synthétiques.

Exemple No. 4

Préparation de l'heptaméthyl-2,3,5,5,6,7,7 tétrahydro-3,5,6,7(s) indacène (2H) one-1

On se place dans les mêmes conditions opératoires que dans l'exemple 1. On utilise les réactifs suivants: 3800 g d'acide polyphosphorique, 260 g d'acide tiglique et 377 g de pentaméthyl-1,1,2,3,3 indane. On obtient, en fin de réaction et d'épuration, 128 g de pentaméthyl-1,1,2,3,3 indane inchangé et recyclable et 278,5 g d'un produit ayant un point d'ébullition situé à 136—156°C sous 13,33 Pa. Le spectre d'absorption infrarouge présente deux bandes principales caractéristiques à 1705 $cm^{-1}$ (très forte) et 1612 $cm^{-1}$ (forte). Le produit obtenu est un mélange d'isomères qui ont été identifiés comme étant de l'heptaméthyl-2,3,5,5,6,7,7 tétrahydro-3,5,6,7(s) indacène(2H)one-1.

Ce produit n'a aucune odeur de musc. Cet essai montre que l'odeur de musc est liée non seulement à la présence d'un radical méthyl en position $\beta$ mais aussi à l'absence de substituant en position $\alpha$.

Exemple No. 5

Préparation de la pentaméthyl-3,5,5,8,8, hexahydro-2,3,5,6,7,8 benz(f) indène (1H) one-1

On applique les mêmes conditions opératoires ues dans l'exemple No. 1. On utilise les réactifs suivants: 2400 g d'acide polyphosphorique, 145 g d'acide crotonique et 240 g de tétraméthyl-1,1,4,4 tétraline.

On obtient, après réaction et séparation, 51 g de tétraméthyl-1,1,4,4 tétraline inchangée et recyclable et 192 g d'un produit ayant un point d'ébullition à 143—144°C sous 13,33 Pa et un point de fusion à 80° C (éthanol).

Le rendement moléculaire est de 74,5% par rapport à la tétraméthyl-1,1,4,4, tétraline transformée.

Le spectre infrarouge présente deux bandes caractéristiques l'une à 1710 $cm^{-1}$ (très forte) et l'autre à 1612 $cm^{-1}$ (forte).

Le spectre de RMN (200 MHz, CDCl3) présente deux singulets à 1,30 et 1,32 et deux singulets ensemble à 1,34 ppm (quatre groupes méthyl géminés); un doublet à 1,40 ppm (J = 7 Hz, méthyl en 3); un singulet à 1,72 ppm (deux groupes CH2 en 6 et 7); deux doublets de doublets centrés à 2,26 ppm (J = 3,6 et 19 Hz) et 2,92 ppm (J = 7,6 et 19 Hz) attribués à un groupe CH2 en 2; un miltiplet complexe entré à 3,40 ppm (J = 3,6; 7,6 et 19 Hz) attribué à un proton tertiaire en 3 et deux singulets à 7,48 et 7,76 ppm (deux protons aromatiques isolés).

Le produit ainsi obtenu a été identifié comme étant de la pentaméthyl-3,5,5,8,8 hexahydro-2,3,5,6,7,8 benz(f) indène (1H) one-1.

Ce produit dégage une odeur de musc, qui est plus faible que celle des produits obtenus dans les exemples 1 à 3 et qui rappelle celle d'autres muscs polycycliques aromatiques.

Le schéma de la réaction qui se produit dans l'exemple No. 5 est le suivant:

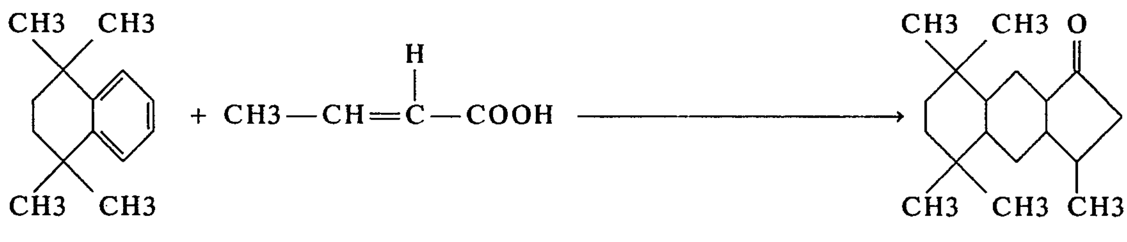

tétraméthyl-1,1,4,4 tétraline + acide crotonique

⟶ pentaméthyl-3,5,5,8,8 hexahydro-2,3,5,6,8, benz(f)indène(1H)one-1.

Exemple No. 6

Préparation de l'hexaméthyl-2,3,5,5,8,8 hexahydro-2,3,5,6,7,8 benz (f) indène (1H) one-1

On applique les mêmes conditions opératoires que dans l'exemple précédent et on remplace l'acide crotonique par l'acide tiglique.

On fait réagir 4300 g d'acide polyphosphorique, 300 g d'acide tiglique et 430 g de tétraméthyl-1,1,4,4 tétraline.

Après séparation des produits de la réaction, on retrouve 113 g de tétraméthyl-1,1,4,4 tétraline qui n'a pas réagi et qui est réutilisable et 328 g d'un produit qui a un point de fusion à 80—83° C (éthanol) et un point d'ébullition à 142°—144° C sous 13,33 Pa. Le spectre infrarouge du produit montre deux bandes principales caractéristiques, l'une située à 1705 ondes $cm^{-1}$, qui est très forte et l'autre située à 1612 ondes $cm^{-1}$ qui est forte.

On identifie le produit obtenu comme un mélange de deux isomères de l'hexaméthyl-2,3,5,5,8,8 hexahydro-2,3,5,6,7,8 benz (f) indène (1H) one-1.

Le rendement moléculaire de la réaction est de 76% par rapport à la tétraméthyl-1,1,4,4, tétraline transformé.

Le produit obtenu dans cet exemple n'a aucune odeur de musc ce qui confirme le résultat négatif décrit dans l'exemple 4.

Les dérivés aromatiques qui ont été soumis dans les exemples décrits à une réaction de cycli-alkylacylation sont des polyméthyl indanes ou des polyméthyl tétralines. Il est précisé que des résultats équivalents ont été obtenus à partir de poly éthyl ou poly propyl indanes ou tétralines et l'on a également obtenu des produits qui dégagent une odeur de musc plus ou moins prononcée.

La durée de la réaction peut être supérieure à la durée totale de l'ordre de 75 minutes qui correspond aux exemples décrits. Cependant, lorsqu'on utilise des acides éthyléniques $\alpha$, $\beta$, une durée de réaction plus longue entraîne la formation de produits de polymérisation et les essais réalisés ont montré qu'une durée totale de réaction comprise entre 45 et 70 minutes est une durée préférentielle.

Des essais ont été également réalisés pour obtenir des produits ne comportant pas de radical méthyle en position $\beta$ mais comportant un radical méthyle en position $\alpha$.

Ces essais ont abouti à des produits ne dégageant aucune odeur de musc utilisable.

Finalement les essais réalisés montrent que l'odeur de musc est liée à la présence d'un noyau pentagonal fixé sur un noyau benzénique et comportant une fonction cétone en positon 1, aucun substituant en position 2 ou $\alpha$ et un seul radical méthyl en position 3 ou $\beta$ et l'invention protège des familles de dérivés substitués des hydrindacénones-1 et des hydro-benz(f) indénones-1 comportant ce groupement caractéristique.

De plus, les essais réalisés ont montré que seuls les dérivés comportant au moins trois substituants alkyle (méthyle, éthyle ou propyle) sur le noyau externe qui ne porte pas la fonction cétone, c'est-à-dire sur l'une des positions 5,5,6,7,8,8 dans le cas d'un noyau hexagonal, présentaient une odeur de musc utilisable dans les parfums.

Finalement l'invention protège à titre de substances nouvelles une famille de produits polycycliques comportant un noyau central benzénique, un deuxième noyau latéral portant une fonction cétone en position 1, aucun substituant en position 2, un seul groupe méthyle en position 3 et un troisième noyau latéral, pentagonal ou hexagonal, comportant au moins trois substituants alkyle.

Grâce à leur odeur de musc caractéristique, ces nouveaux produits synthétiques sont utilisables comme parfums ou comme composants d'une composition de parfums.

Par composition de parfum, on désigne un mélange d'un ou plusieurs produits dégageant un parfum qui peuvent être mélangés à diverses substances auxiliaires destinées à fixer ou à stabiliser le parfum. De plus, le mélange peut être dissous dans un solvant approprié ou mélangé avec un substrat gras ou pulvérulent et/ou à toutes sortes de produits destinés à faciliter l'application du parfum sur la peau, sur les cheveux etc. ...

Les produits auxiliaires entrant dans les compositions de parfums sont bien connus de l'homme de l'art.

A titre d'exemple non limitatif, on peut citer les savons, les détergents, les cosmétiques tels que des crèmes, pommades, eaux de toilette, lotions avant ou après rasage, les produits capillaires, les déodorants etc. ...

Les produits selon l'invention peuvent être mélangés à d'autres parfums qui peuvent être des produits naturels ou des parfums synthétiques.

Les compositions de parfums contenant des composés selon l'invention peuvent être dissoutes dans des solvants par exemple dans l'éthanol, l'éther mono-éthylique de diéthylène glycol, le myristate d'isopropyle ou les esters de l'alcool $\beta$-phényléthylique, cette énumération n'étant pas limitative. La quantité de musc synthétique selon l'invention entrant dans un parfum ou dans une composition de parfum varie beaucoup selon le produit dans lequel le parfum est incorporé, selon la nature et la quantité des autres constituants de la composition et selon l'invensité de l'odeur que l'on désire obtenir.

Une proportion en poids très faible, de l'ordre de 0,01% suffit à conférer à une composition de parfum une odeur de musc perceptible.

On donnera ci-après, sans aucun caractère limitatif, des exemples de composition de parfums contenant des muscs artificiels selon l'invention.

Exemple No. 7

Composition de parfums de type floral destiné à être utilisé dans des cosmétiques

| Proportion en poids | Produit |
|---|---|
| 90 | essence d'ylang-ylang |
| 150 | alcool phényléthylique |
| 80 | géraniol |
| 80 | cytronellol |
| 30 | héliotropine |
| 50 | linalol |
| 100 | α-méthylione |
| 100 | salicylate d'hexyle |
| 50 | acétate de linalyle |
| 20 | undécalactone à 10% dans le dipropylène glycol (DPG) |
| 60 | essence de camomille du Maroc à 10% dans le DPG |
| 35 | acétate de benzyle |
| 5 | acétate du diméthylbenzylcarbinol |
| 150 | hexaméthyl-3,5,5,6,7 tétrahydro-3,5,6,7 \|s\| indacène (2H) one-1 |
| TOTAL: 1000 | |

Exemple No. 8

Composition de parfum de type floral destinée à des bains moussants

| Proportions en poids | Produit |
|---|---|
| 20 | acétate d'isobornyle |
| 100 | aldéhyde $\alpha$-hexylcinnamique |
| 50 | linalol |
| 100 | méthylionone |
| 50 | salicylate d'éthyle |
| 70 | terpinéol |
| 100 | alcool phényléthylique |
| 20 | acétate de benzyle |
| 30 | essence de lavandin Abrialis |
| 10 | essence de patchouly |
| 50 | géraniol |
| 50 | citronellol |
| 50 | acétate de cédrényle |
| 50 | acétate d'isoeugényle |
| 50 | acétate de pipéronyle |
| 200 | hexaméthyl-3,5,5,6,7,7 tétrahydro-3,5,6,7 \|s\| indacène (2H) one-1 |
| TOTAL: 1000 | |

Exemple No. 9

Composition de parfum de type floral à utiliser dans les détergents

| Proportions en poids | Produit |
|---|---|
| 20 | héliotropine |
| 90 | alcool phényléthylique |
| 50 | citronellol |
| 30 | acétate de benzyle |
| 5 | citral |
| 40 | géraniol |
| 90 | aldéhyde α-hexylcinnamique |
| 75 | salicylate de benzyle |
| 5 | α-ionone |
| 500 | hydroxycitronellal |
| 20 | alcool phénylpropylique |
| 10 | (hydroxy-4 phényl)-4 butanone-2 à 1% dans le dipropylène glycol (DPG) |
| 10 | aubépine à 10% dans le DPG |
| 10 | aldéhyde phénylacétique à 10% dans le DPG |
| 10 | nonadiène-2,6 al à 1% dans le DPG |
| 10 | essence d'ylang-ylang |
| 25 | hexaméthyl-3,5,5,6,7,7 tétrahydro-3,5,6,7 \|s\| indacène (2H) one-1 |
| TOTAL: 1000 | |

Exemple No. 10

Composition de parfum de type fougère destinée aux savons

| Proportions en poids | Produit |
|---|---|
| 20 | essence de patchouly |
| 10 | essence de lavande |
| 80 | géraniol |
| 80 | acétate de terpényle |
| 100 | α-terpinéol |
| 50 | absolue de mousse d'arbre |
| 20 | coumarine |
| 10 | dihydromyrcénol |
| 40 | acétyl-1 éthano-7-10 triméthyl-4-4,7octaline-1(9) |
| 340 | alcool phényléthylique |
| 100 | linalol |
| 100 | essence de bergamote sans furocoumarines |
| 50 | hexaméthyl-3,5,5,6,7,7 tétrahydro-3,5,6,7 \|s\| indacène (2H) one-1 |
| TOTAL: 1000 | |

Exemple No. 11

Composition de parfum du type fougère destiné aux extraits ou eau de toilette alcooliques

| Proportions en poids | Produit |
|---|---|
| 10 | essence de rose turque |
| 40 | essence d'ylang-ylang |
| 100 | essence de patchouly |
| 150 | essence de géranium Bourbon |
| 250 | essence de lavandin Abrialis |
| 100 | absolue de mousse de chêne |
| 50 | eugénol 100% |
| 50 | salicylate de benzyle |
| 50 | salicylate d'amyle |
| 50 | coumarine |
| 30 | $\alpha$-méthyl-p-tert-butylhydrocinnamaldéhyde |
| 50 | (hydroxy-4 méthyl-4 pentyl)-4 cyclohex-3)ènecarboxaldéhyde |
| 20 | alcool phényléthylique |
| 50 | hexaméthyl-3,5,5,6,7,7 tétrahydro-3,5,6,7 \|s\| indacène (2H) one-1 |
| TOTAL: 1000 | |

Les exemples qui précèdent se réfèrent à des compositions de parfums contenant toutes de l'hexaméthyl 3,5,5,6,7,7 hexahydro 1,2,3,5,6,7 [s] indacène-1 (2H)one 1 car parmi les dérivés synthétisés, c'est celui qui a l'ordeur de musc la plus prononcé. Toutefois, ce dérivé peut être remplacé par les autres muscs synthétiques faisant l'objet de l'invention et entrant dans la définition donnée par la formule générale.

La proportion de dérivé variera selon les propriétés olfactives du dérivé utilisé.

## Revendications

1. Produits nouveaux dérivés substitués polyalkylés des indanones-1, caractérisés en ce qu'ils appartiennent à la famille de substances synthétiques définies par la formule développée:

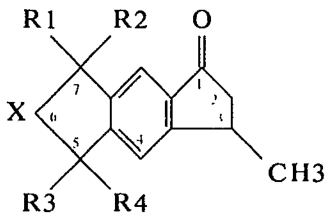

dans laquelle R1, R2, R3, R4 désignent séparément un hydrogène ou un radical alkyle ayant un à trois atomes de carbone, dans laquelle X représente un groupe $-CHR_5-$ ou un groupe $-CHR_5-CHR_6-$, dans lesquels R5, R6, représentent séparément un hydrogène ou un radical méthyle et dans laquelle au moins trois des radicaux R1, R2, R3, R4, R5 et R6 sont des radicaux alkyles, lesquels dérivés comportent tous une fonction cétone en position 1, aucun substituant en position 2, un seul groupe méthyl en position 3 et un noyau pentagonal ou hexagonal, symétrique du noyau portant la fonction cétone, comportant au moins trois substituants alkyl.

2. Produits nouveaux selon la revendication 1, dérivés polyalkylés de l'hydro(s) indacène (2H) one-1, caractérisés en ce qu'ils appartiennent à la famille de substances définies par la formule développée:

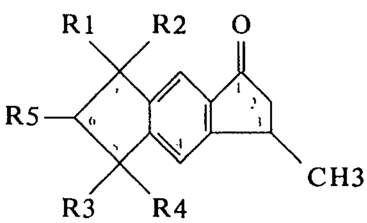

dans laquelle R1, R2, R3, R4 représentent un hydrogène ou un radical alkyle ayant un à trois atomes de carbone, R5 représente un hydrogène ou un radical méthyle et trois au moins des radicaux R1, R2, R3, R4, R5 sont des radicaux alkyle.

3. Produits nouveaux selon la revendication 1 dérivés polyalkylés de l'hydro-benz(f)indène (1H)one-1, caractérisés en ce qu'ils appartiennent à la famille de substances définies par la formule développée:

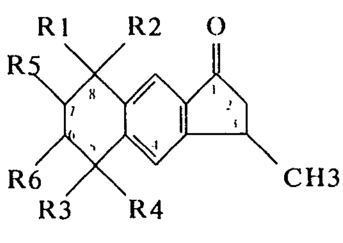

dans laquelle R1, R2, R3, R4 représentent un hydrogène ou un radical alkyle ayant un à trois atomes de carbone, R5, R6 représentent un hydrogène ou un radical méthyle et trois au moins des radicaux R1, R2, R3, R4, R5, R6 sont des radicaux alkyle.

4. Produit nouveau selon l'une quelconque des revendications 1 et 2 dérivé substitué de l'hydro(s)indacène(2H)one-1, caractérisé en ce qu'il répond à la dénomination hexaméthyl-3,5,5,6,7,7 tétrahydro-3,5,6,7(s)-indacène(2H) one-1, qu'il a un point d'ébullition à 140—142°C sous 13,33 Pa un indice de réfraction $n_{20}^{D} = 1,551$ et qu'il est représenté par la formule développée:

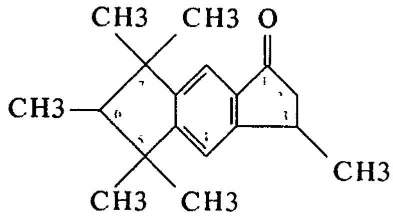

5. Produit nouveau selon l'une quelconque des revendications 1 et 2 dérivé substitué de l'hydro(s)indacène(2H) one-1, caractérisé en ce qu'il répond à la dénomination pentaméthyl-3,5,5,7,7, tétrahydro-3,5,6,7(s) indacène(2H)one-1, qu'il a un point d'ébullition de 136—138° C sous 13,33 Pa et un point de fusion de 83—84° C et qu'il est représenté par la formule développée:

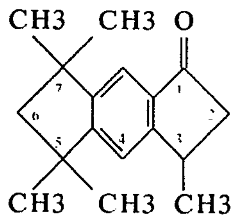

6. Produit nouveau selon la revendication 3 dérivé substitué de l'hydro benz(f)indène(1H)one-1, caractérisé en ce qu'il répond à la dénomination pentaméthyl-3,5,5,8,8, hexahydro-2,3,5,6,7,8 benz(f)indène(1H)one-1, qu'il a un point d'ébullition à 143—144° C sous 13,33 Pa et un point de fusion de 80° C et qu'il est représenté par la formule développée:

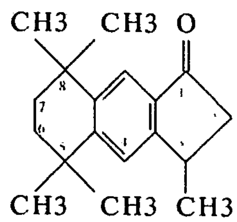

7. Procédé de synthèse de dérivés substitués polyalkylés d'indanones-1 comportant un seul radical méthyl en position 3, caractérisé en ce que l'on soumet un dérivé aromatique pris dans le groupe des hydrocarbures polyalkylés indaniques ou tétraliniques à une réaction de cycli-alkylacylation par un acide éthylénique -$\alpha,\beta$ ou par un ester méthylique ou éthylique d'un acide éthylénique -$\alpha,\beta$ en présence d'acide polyphosphorique à une température comprise entre 90° C et 150° C et, de préférence, entre 120° C et 135° C, pendant une durée comprise entre 30 minutes et 180 minutes et, de préférence, entre 45 et 70 minutes.

8. Procédé selon la revendication 7 de synthèse de l'hexaméthyl-3,5,5,6,7,7, tétrahydro-3,5,6,7(s) indacène(2H)one-1, caractérisé en ce que l'on fait réagir du pentaméthyl-1,1,2,3,3 indane avec de l'acide crotonique ou un ester méthylique ou éthylique de l'acide crotonique dans de l'acide polyphosphorique.

9. Procédé selon la revendication 7 de synthèse de pentaméthyl-3,5,5,7,7, tétrahydro-3,5,6,7(s) indacène(2H) one-1, caractérisé en ce que l'on fait réagir du tétraméthyl-1,1,3,3, indane avec de l'acide crotonique ou un ester méthylique ou éthylique de l'acide crotonique dans de l'acide polyphosphorique.

10. Procédé selon la revendication 7 de synthèse de pentaméthyl-3,5,5,8,8, hexahydro-2,3,5,6,7,8(1H)benz(f)indène (1H)one-1, caractérisé en ce que l'on fait réagir de la tétramethyl-1,1,4,4, tétraline avec de l'acide crotonique ou avec un ester méthylique ou éthylique de l'acide crotonique en présence d'acide polyphosphorique.

11. Parfum ou composition de parfum, caractérisée en ce qu'elle contient au moins une substance synthétiques prise dans les familles définies par les revendication 1, 2 et 3.

12. Parfum ou composition de parfum, caractérisée en ce qu'elle contient au moins une substance synthétique prise dans le groupe composé de:

— l'hexaméthyl)-3,5,5,6,7,7,tétrahydro-3,5,6,7(s)indacène(2H)one-1;
— le pentaméthyl-3,5,5,7,7,tétrahydro-3,5,6,7(s)indacène(2H)one-1 et
— le pentaméthyl-3,5,5,8,8,hexahydro-2,3,5,6,7,8 benz(f)indène(1H)one-1.

**Patentansprüche**

1. Neue Produkte, nämlich substituierte, polyalkylierte Indan-1-on-Derivate, dadurch gekennzeichnet, daß sie zur Familie von synthetischen Substanzen gehören, welche durch die Strukturformel

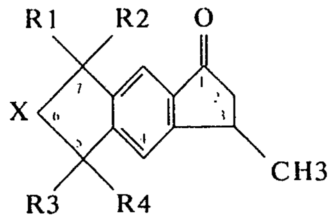

definiert sind, worin R1, R2, R3, R4 jeweils Wasserstoff oder einen Alkylrest mit 1 bis 3 Kohlenstoffatomen bedeuten, X für eine Gruppe —CHR5 — oder eine Gruppe —CHR5 — CHR6 —, in denen R5, R6 jeweils Wasserstoff oder einen Methylrest bedeuten, steht, worin mindestens drei der Reste R1, R2, R3, R4, R5 und R6 Alkylreste darstellen, welche Derivate alle eine Ketofunktion in Position 1, keinen Substituenten in Funktion 2, eine einzige Methylgruppe in Position 3 und einen pentagonalen oder hexagonalen, zu dem die Ketofunktion tragenden Ring symmetrischen Ring aufweisen, welcher mindestens drei Alkylsubstituenten aufweist.

2. Neue Produkte nach Anspruch 1, nämlich Polyalkylderivate von Hydro(s)-(2H)-indacen-1-on, dadurch gekennzeichnet, daß sie zur Familie von Substanzen gehören, welche durch die Strukturformel

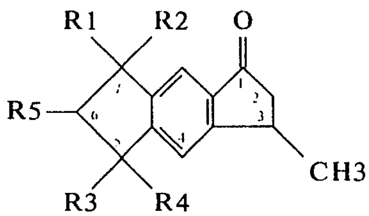

definiert ist, worin R1, R2, R3, R4 Wasserstoff oder einen Alkylrest mit 1 bis 3 Kohlenstoffatomen darstellen, R5 für Wasserstoff oder einen Methylrest steht und mindestens drei der Reste R1, R2, R3, R4, R5 Alkylradikale sind.

3. Neue Produkte nach Anspruch 1, nämlich Polyalkylderivate von Hydro-(1H)-benz[f]iden-1-on, dadurch gekennzeichnet, daß sie zur Familie von Substanzen gehören, welche durch die Strukturformel

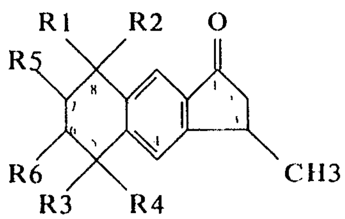

definiert sind, worin R1, R2, R3, R4 Wasserstoff oder einen Alkylrest mit 1 bis 3 Kohlenstoffatomen darstellen, R5 und R6 für Wasserstoff oder ein Methylradikal stehen und mindestens drei der Reste R1, R2, R3, R4, R5 und R6 Alkylradikale bedeuten.

4. Neues Produkt nach einem der Ansprüche 1 und 2, nämlich ein substituiertes Derivat von Hydro(s)-(2H)-indacen-1-on, dadurch gekennzeichnet, daß es die Bezeichnung 3,5,5,6,7,7-Hexamethyl-3,5,6,7(s)-tetrahydro-(2H)-indacen-1-on hat, daß es einen Kochpunkt von 140—142°C unter 13,33 Pa, einen Brechungsindex $n_{20}^{D}$ = 1,551 aufweist, und daß es durch die Strukturformel

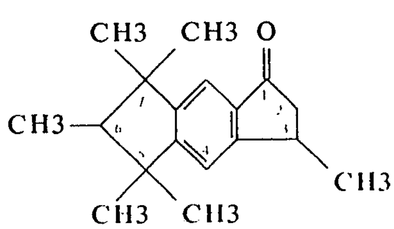

repräsentiert wird.

5. Neues Produkt nach einem der Ansprüche 1 und 2, nämlich ein substituiertes Derivat von Hydro(s)-(2H)-indacen-1-on, dadurch gekennzeichnet, daß es die Bezeichnung 3,5,5,7,7-Pentamethyl-3,5,6,7(s)-tetrahydro-(2H)-indacen-1-on hat, daß es einen Kochpunkt von 136—138° C unter 13,33 Pa und einen Schmelzpunkt von 83—84° C aufweist, und daß es durch die Strukturformel

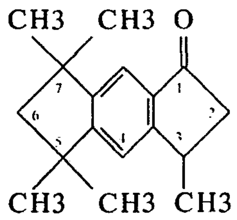

repräsentiert wird.

6. Neues Produkt nach Anspruch 3, nämlich ein substituiertes Derivat von Hydro-(1H)-benz[f]iden-1-on, dadurch gekennzeichnet, daß es die Bezeichnung 3,5,5,8,8-Pentamethyl-2,3,5,6,7,8-hexahydro-(1H)-benz[f]iden-1-on hat, daß es einen Kochpunkt von 143—144° C unter 13,33 Pa und einen Schmelzpunkt von 80° C aufweist, und daß es durch die Strukturformel

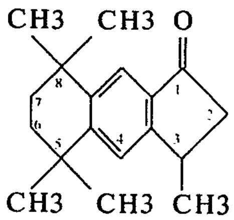

repräsentiert wird.

7. Verfahren zur Synthese von substituierten, polyalkylierten Indan-1-on-Derivaten, welche ein einziges Methylradikal in Position 3 tragen, dadurch gekennzeichnet, daß man ein aromatisches Derivat aus der Gruppe der polyalkylierten Indan- oder Tetralin-Kohlenwasserstoffe einer Cyclisierung-Alkylacylierung mit einer $\alpha,\beta$-äthylenischen Säure oder mit einem Methyl- oder Äthylester einer $\alpha,\beta$-äthylenischen Säure in Gegenwart von Polyphosphorsäure bei einer Temperatur zwischen 90° C und 150° C, vorzugsweise zwischen 120° C und 135° C, während eines Zeitraums zwischen 30 min und 180 min, vorzugsweise zwischen 45 und 70 min, unterwirft.

8. Verfahren nach Anspruch 7 zur Synthese von 3,5,5,6,7,7-Hexamethyl-3,5,6,7(s)-tetrahydro-(2H)-indacen-1-on, dadurch gekennzeichnet, daß man 1,1,2,3,4-Pentamethyl-indan mit Crotonsäure oder einem Methyl- oder Äthylester der Crotonsäure in Polyphosphorsäure zur Umsetzung bringt.

9. Verfahren nach Anspruch 7 zur Synthese von 3,5,5,7,7-Pentamethyl-3,5,6,7(s)-tetrahydro-(2H)-indacen-1-on, dadurch gekennzeichnet, daß man 1,1,3,3-Tetramethyl-indan mit Crotonsäure oder einem Methyl- oder Äthylester der Crotonsäure in Polyphosphorsäure zur Umsetzung bringt.

10. Verfahren nach Anspruch 7 zur Synthese von 3,5,5,8,8-Pentamethyl-2,3,5,6,7,8-hexahydro-(1H)-benz[f]iden-1-on, dadurch gekennzeichnet, daß man 1,1,4,4-Tetramethyl-tetralin mit Crotonsäure oder einem Methyl- oder Äthylester der Crotonsäure in Gegenwart von Polyphosphorsäure zur Umsetzung bringt.

11. Parfum oder Parfumzusammensetzung, dadurch gekennzeichnet, daß mindestens eine synthetische Substanz aus den Familien gemäß den Ansprüchen 1, 2 und 3 enthalten ist.

12. Parfum oder Parfumzusammensetzung, dadurch gekennzeichnet, daß mindestens eine synthetische Substanz aus der Gruppe bestehend aus

— 3,5,5,6,7,7-Hexamethyl-3,5,6,7(s)-tetrahydro-(2H)-indacen-1-on;
— 3,5,5,7,7-Pentamethyl-3,5,6,7(s)-tetrahydro-(2H)-indacen-1-on und
— 3,5,5,8,8-Pentamethyl-2,3,5,6,7,8-hexahydro-(1H)-benz[f]iden-1-on enthalten ist.

**Claims**

1. New polyalkyl substituted dervatives of indan-1-ones, characterized in that they belong to the family of synthetic substances, defined by the structural formula:

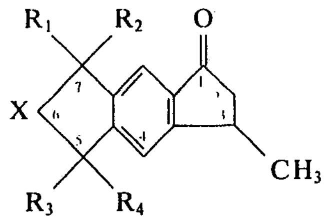

in which $R_1$, $R_2$, $R_3$, $R_4$ denote, separately, a hydrogen or an alkyl radical having one to three atoms of carbon, in which X represents a $-CHR_5$ group or a $-CHR_5 - CHR_6 -$ group, in which $R_5$, $R_6$ represent, separately, a hydrogen or a methyl radical and in which at least three of the radicals $R_1$, $R_2$, $R_3$, $R_4$, $R_5$ and $R_6$ are alkyl radicals, which derivatives all comprise a ketone function in 1 position, no substitute in 2 position, one methyl group in 3 position and a five-membered or six-membered ring, symmetrical with respect to the ring bearing the ketone function, comprising at least three alkyl substitutes.

2. New products according to claim 1, polyalkyl dervatives of hydro(s) indacen(2H)-one, characterized in that they belong to the family of synthetic substances defined by structural formula:

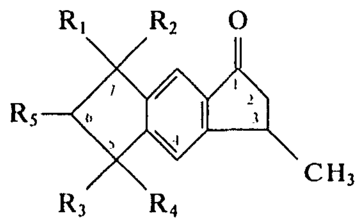

in which $R_1$, $R_2$, $R_3$, $R_4$ represent a hydrogen or an alkyl radical having one to three atoms of carbon, $R_5$ represents a hydrogen or a methyl radical and at least three of the radicals $R_1$, $R_2$, $R_3$, $R_4$, $R_5$ are alkyl radicals.

3.New products according to claim 1, polyalkyl derivatives of hydro-benz(f) inden(1H)-1-one, characterized in that they belong to the family of synthetic substances defined by structural formula:

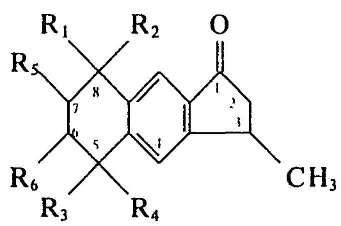

in which $R_1$, $R_2$, $R_3$, $R_4$ represent a hydrogen or an alkyl radical having one to three carbon atoms, $R_5$, $R_6$ represent a hydrogen or a methyl radical and at least three of the radicals $R_1$, $R_2$, $R_3$, $R_4$, $R_5$, $R_6$ are alkyl radicals.

4. A new product according to any one of claims 1 and 2, substituted derivative of hydro(s) indacen(2H)-1-one, characterized in that it corresponds to the nomenclature 3,5,5,6,7,7-hexamethyl 3,5,6,7-tetrahydro(s)-indacen(2H)-1-one, it has a boiling point of 140—142°C under 0.1 mm Hg, a refraction index of $n_{20}^{D} = 1.551$ and represented by structural formula:

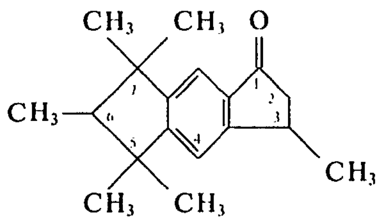

5. A new product according to any one of claims 1 and 2, substituted derivative of hydro(s) indacen(2H)-1-one, characterized in that it corresponds to the nomenclature 3,5,5,7,7-pentamethyl 3,5,6,7-tetrahydro(s) indacen(2H)-1-one, it has a boiling point of 136—138°C under 0.1 mm Hg and a melting point of 83—84°C and represented by structural formula:

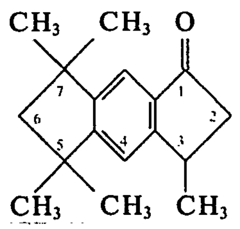

6. New product according to claim 3, substituted derivative of hydro benz(f)inden(1H)-1-one, characterized in that is corresponds to the nomenclature 3,5,5,8,8-pentamethyl 2,3,5,6,7,8-hexahydro benz(f) inden(1H)-1-one, it has a boiling point of 143—144° C under 0.1 mm Hg and a melting point at 80° C and that it is represented by structural formula:

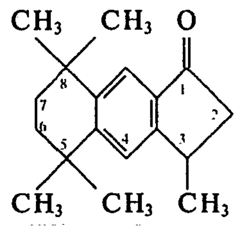

7. A process for synthesizing polyalkyl substituted derivatives of indan-1-ones comprising one methyl radical in 3 position, characterized in that an aromatic derivative selected from the group of polyalkyl indanic or tetralinic hydrocarbons is subjected to a reaction of cycli-alkylacylation by an $\alpha,\beta$-ethylene acid or by a methyl or ethyl ester of an $\alpha,\beta$-ethylene acid in the presence of polyphosphoric acid at a temperature of between 90° C and 150° C and, preferably, between 120° C and 135° C, for a duration of between 30 minutes and 180 minutes and, preferably, between 45 and 70 minutes.

8. A process according to claim 7 for synthesizing 3,5,5,6,7,7-hexamethyl 3,5,6,7-tetrahydro(s) indacen(2H)-1-one, characterized in that 1,1,2,3,3-pentamethyl indane is reacted with crotonic acid or a methyl or ethyl ester of crotonic acid in polyphosphoric acid.

9. A process according to claim 7 for synthesizing 3,5,5,7,7-pentamethyl 3,5,6,7-tetrahydro(s) indacen(2H)-1-one, characterized in that 1,1,3,3-tetramethyl indane ist reacted with crotonic acid or a methyl or ethyl ester of crotonic acid in polyphosphoric acid.

10. A process according to claim 7 for synthesizing 3,5,5,8,8-pentamethyl 2,3,5,6,7,8-hexahydro(1H)benz(f) inden(1H)-1-one, characterized in that 1,1,4,4-tetramethyl tetraline is reacted with crotonic acid or with a methyl or ethyl ester of crotonic acid in the presence of polyphosphoric acid.

11. A perfume or perfume composition, characterized in that it comprises at least one synthetic substance selected from the products defined in claims 1, 2 or 3.

12. A perfume or perfume composition, characterized in that it comprises at least one synthetic substance selected from the group composed of:

— 3,5,5,6,7,7-hexamethyl 3,5,6,7-tetrahydro(s) indacen(2H)-1-one;
— 3,5,5,7,7-pentamethyl 3,5,6,7-tetrahydro(s) indacen(2H)-1-one, and
— 3,5,5,8,8-pentamethyl 2,3,5,6,7,8-hexahydro benz(f) inden(1H)-1-one.

Fig.1

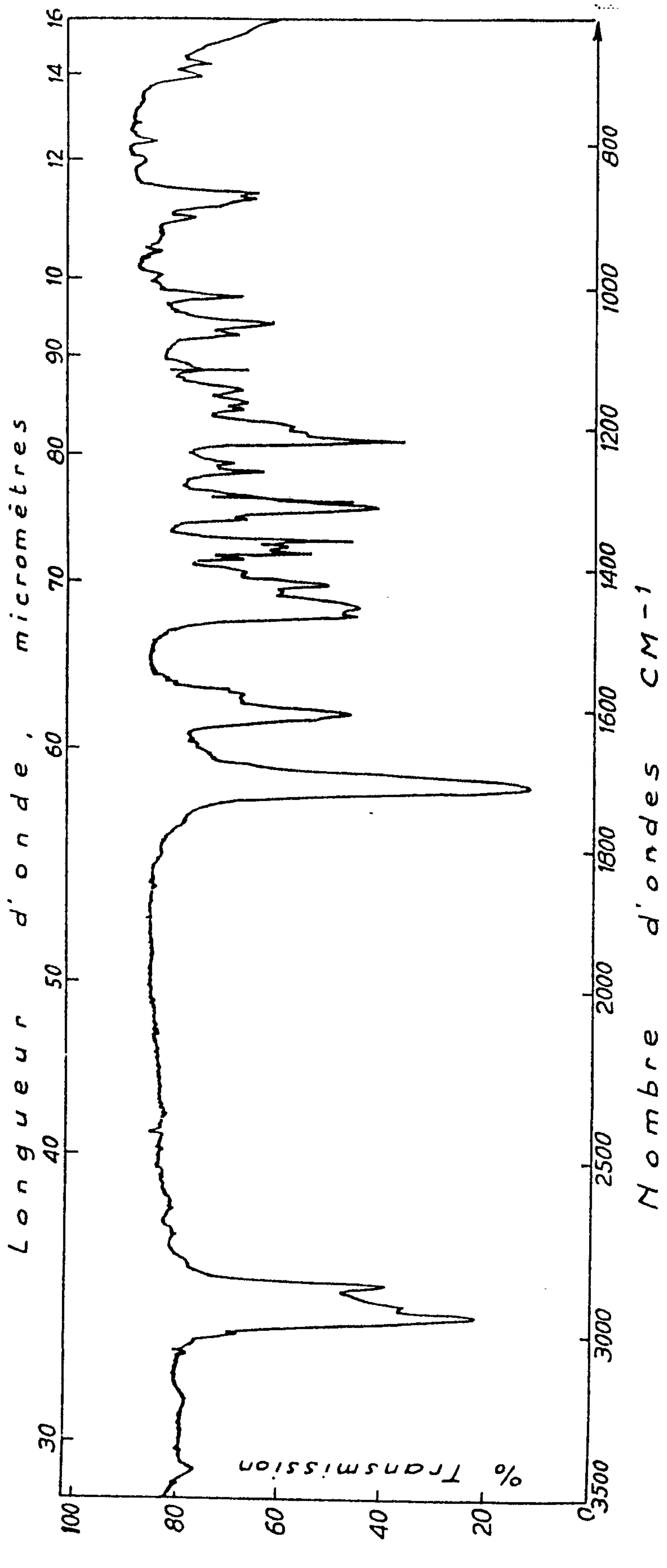
Longueur d'onde, micromètres
Nombre d'ondes CM-1
% Transmission
100
80
60
40
20
0
30
40
50
60
70
80
90
10
12
14
16
3500
3000
2500
2000
1800
1600
1400
1200
1000
800

FIG. 2

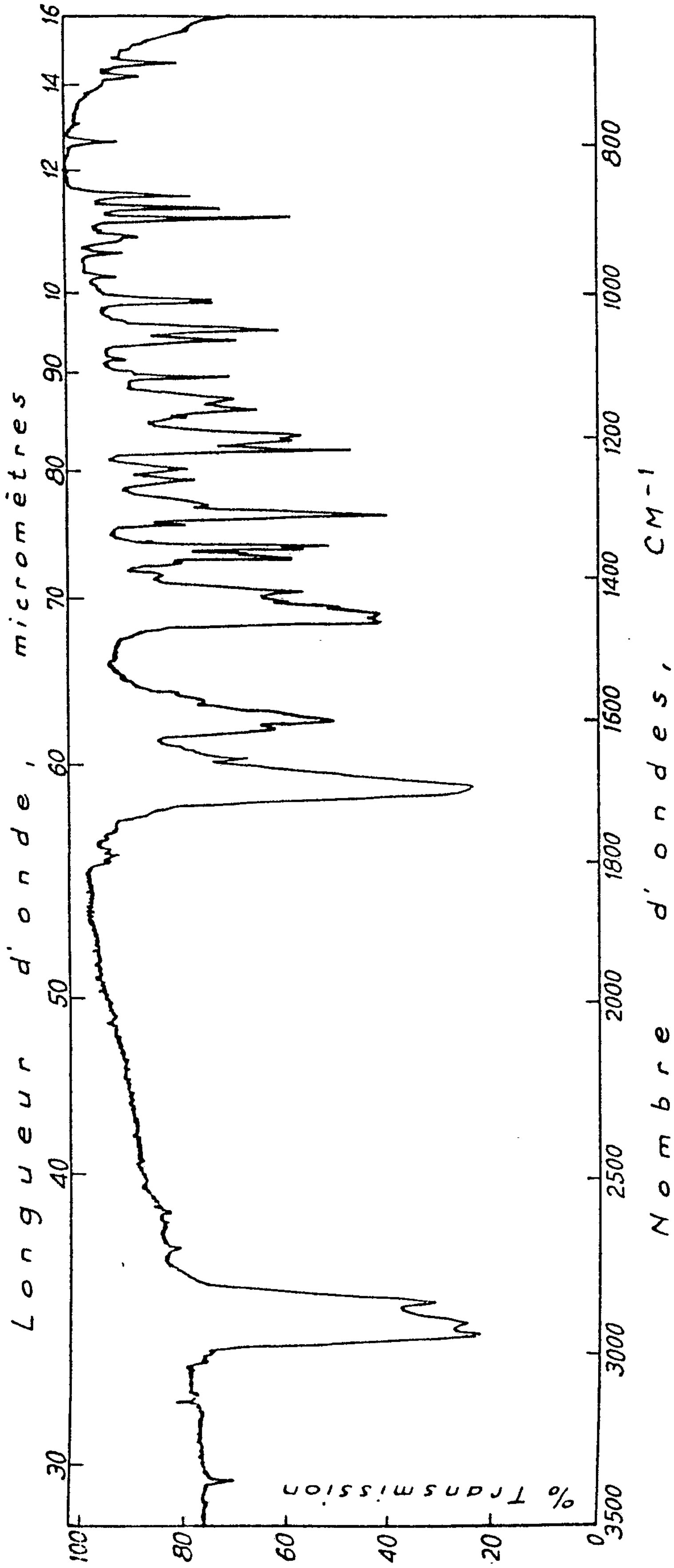
Longueur d'onde, micromètres
Nombre d'ondes, CM-1
% Transmission
30
40
50
60
70
80
90
10
12
14
16
3500
3000
2500
2000
1800
1600
1400
1200
1000
800
100
80
60
40
20
0

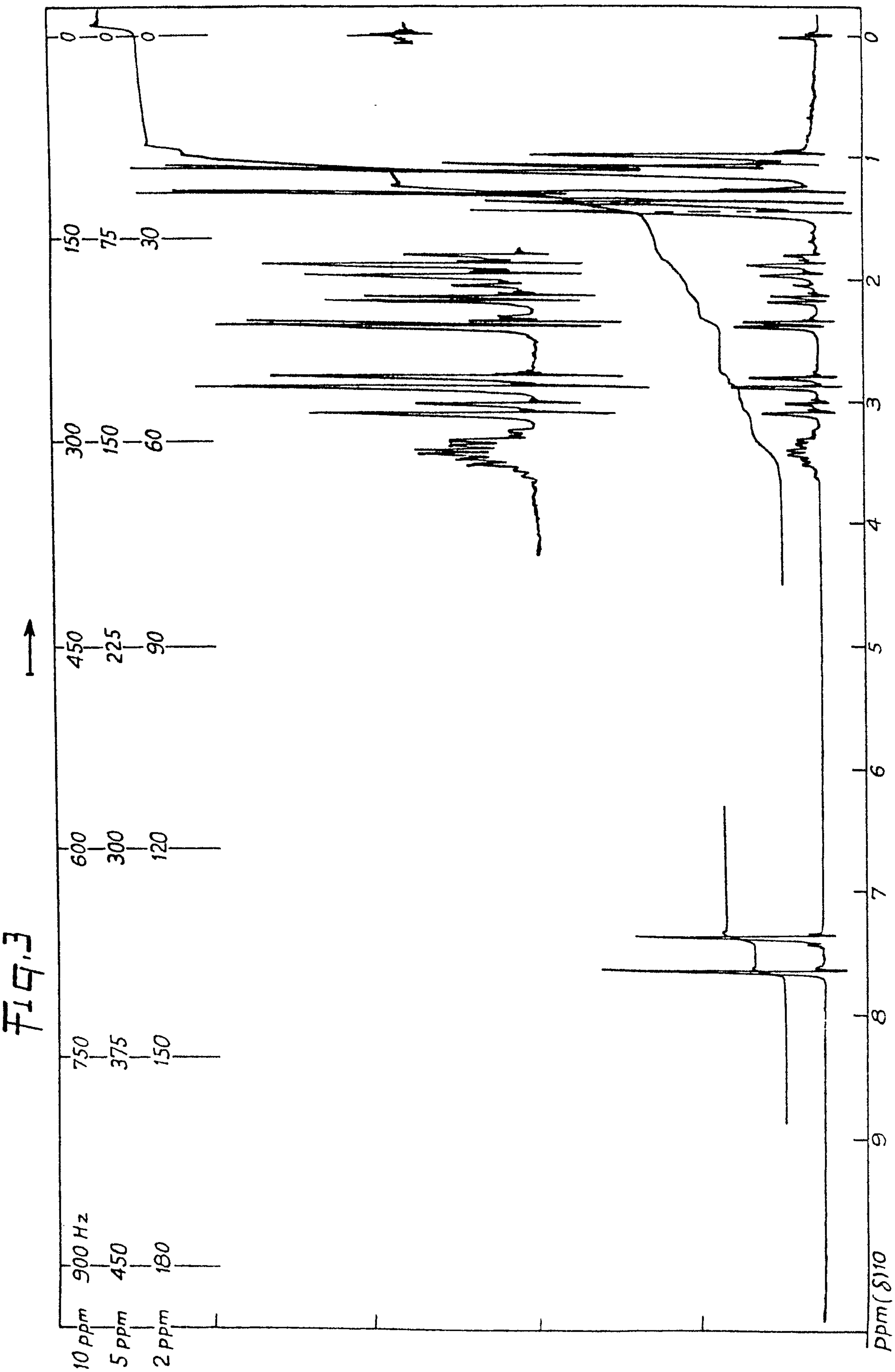
900 Hz
450
180
750
375
150
600
300
120
450
225
90
300
150
60
150
75
30
0
10 ppm
5 ppm
2 ppm
ppm (δ) 10
9
8
7
6
5
4
3
2
1
0

Fig. 3